# EUROPEAN PATENT SPECIFICATION

(11) **EP 4 525 698 B1**
(45) Date of publication and mention of the grant of the patent: **09.12.2026**
(21) Application number: 23732199.7
(22) Date of filing: 22.05.2023
(51) Int. Cl.: A61B 5/07

(54) **RESONANT CIRCUIT-BASED VASCULAR MONITORS AND RELATED SYSTEMS AND METHODS**
AUF SCHWINGKREIS BASIERENDE GEFÄSSMONITORE SOWIE ENTSPRECHENDE SYSTEME UND VERFAHREN
MONITEURS VASCULAIRES BASÉS SUR UN CIRCUIT RÉSONANT ET SYSTÈMES ET PROCÉDÉS ASSOCIÉS

(30) Priority: 20.05.2022 US 202263344409 P
(43) Date of publication of application: 26.03.2025
(73) Proprietor: Foundry Innovation & Research 1, Ltd., Dublin 2 (IE)
(72) Inventor: MARTIN, Pablo, Dublin, 2 (IE); KELLY, Michael, Dublin, 2 (IE); WETTERLING, Friedrich, Dublin, 2 (IE); MCDONALD, Jack, Dublin, 2 (IE); SWEENEY, Fiachra M., Dublin, 2 (IE)
(74) Representative: Tomkins & Co
(86) International application number: PCT/IB2023/055245
(87) International publication number: WO 2023/223301

(56) References cited:
- WO-A1-2018/102435
- WO-A1-2019/232213
- WO-A1-2021/094980
- US-A1- 2007 247 138
- US-A1- 2019 150 882
- US-B2- 11 206 992

## Description

### RELATED APPLICATIONS

The present application claims priority to U.S. Provisional Patent Application No. 63/344,409, filed May 20, 2022, entitled "Resonant Circuit-Based Monitors and Related Systems and Methods", q.v..

### FIELD

The present disclosure relates to improvements in wireless vascular monitors, in particular, resonant circuit-based vascular monitors and related systems and methods.

### BACKGROUND

Resonant circuit (RC)-based sensors are sensors that deliver a change in resonant frequency as a result of a change in a physical parameter in the surrounding environment, which change causes the resonant frequency produced by the circuit within the device to change. The change in resonant frequency, which may be detected as a "ring-back" signal when the circuit is energized, indicates the sensed parameter or change therein. As is well-known, a basic resonant circuit includes an inductance and a capacitance. In most available RC sensing devices, the change in resonant frequency results from a change in the capacitance of the circuit. The plates of a capacitor moving together or apart in response to changes in pressure, thus providing a pressure sensor, is a well-known example of such a device. Less commonly, the change in resonant frequency is based on a change in the inductance of the circuit.

The present Applicant has filed a number of patent applications disclosing new RC monitoring devices using variable inductance for monitoring intravascular dimensions and determining physiological parameters such as patient fluid state based thereon. See, for example, PCT/US2017/063749, entitled "Wireless Resonant Circuit and Variable Inductance Vascular Implants for Monitoring Patient Vasculature and Fluid Status and Systems and Methods Employing Same", filed November 29, 2017 (Pub. No. WO2018/102435) and PCT/US2019/034657, entitled "Wireless Resonant Circuit and Variable Inductance Vascular Monitoring Implants and Anchoring Structures Therefore", filed May 30, 2019 (Pub. No. WO2019/232213), which disclose a number of different embodiments and techniques related to such devices. WO 2021/094980 A1 discloses resonant circuit-based vascular monitors and related systems and methods. US 2007/247138 A1 discloses communicating with an implanted wireless sensor.

Notwithstanding the advances in the art represented by these prior disclosures, improvements in control and signal processing for such devices can still be made. The present disclosure thus offers solutions to some unique problems described herein, which have been encountered only after introduction and testing of the aforementioned new RC monitoring devices.

### SUMMARY OF THE DISCLOSURE

In one implementation, the present disclosure is directed to a method for controlling a wireless resonant circuit sensor, the sensor including a variable inductance coil that changes resonant frequency in response to a change in a monitored physical parameter and produces a ring-back signal with a signal magnitude correlated to the physical parameter when energized. The method includes outputting an excitation signal selected to produce the ring-back signal from the sensor; receiving the ring-back signals from the sensor at a receiving amplifier; comparing the magnitude of the sensor ring-back signal to a dynamic range of the receiving amplifier; reducing receiving amplifier gain when compared magnitude is at or exceeds a magnitude dynamic range of the receiving amplifier. If the amplitude is at the limit of the dynamic range, then the receiver gain can be reduced to bring the system back into its linear range.

In another implementation, the present disclosure is directed to a method for characterizing a resonant circuit sensor to correlate sensor output to a measured physical parameter, wherein the sensor comprises a variable inductance coil that changes resonant frequency in response to a change in the physical parameter by producing, when energized, a ring-back signal having a signal magnitude correlateable to the physical parameter. The method includes determining physical parameter value versus signal magnitude data over a range of parameter values and signal magnitudes for at least one of the sensors prior to placement in a patient; and creating a signal magnitude characterization curve for the at least one sensor by plotting a curve with the signal magnitude data using curve fitting or interpolation techniques.

In yet another implementation, the present disclosure is directed to a method for controlling a wireless resonant circuit sensor, the sensor including a variable inductance coil that changes resonant frequency in response to a change in a monitored physical parameter and produces a ring-back signal at a frequency or magnitude correlated to the physical parameter when energized. The method includes outputting a sensor energizing signal at an initial transmit frequency; receiving the ring-back signal at a ring-back frequency from the sensor in response to the sensor energizing signal; determining a difference between the transmit frequency and the ring-back frequency; periodically repeating the outputting, receiving and determining while the difference between the transmit frequency and ring-back frequency is below a predetermined threshold; changing the sensor energizing signal transmit frequency to a new transmit frequency matching the ring-back frequency of a last received ring-back signal when the difference meets or exceeds the predetermined threshold; and periodically repeating the outputting at the new transmit frequency and thereafter repeating the receiving and determining.

### BRIEF DESCRIPTION OF THE DRAWINGS

For the purpose of illustrating the invention, the drawings show aspects of one or more embodiments of the invention. However, it should be understood that the present invention is not limited to the precise arrangements and instrumentalities shown in the drawings, wherein:
FIG. 1 is a schematic system overview of an embodiment of a wireless vascular monitoring system employing a resonant circuit-based sensor implant.
FIG. 2 is a block diagram of an embodiment of a control system for wireless vascular monitoring systems disclosed herein.
FIGS. 3A, 3B and 3C illustrate signals obtained in *in vivo* pre-clinical experiments using a prototype RC-WVM system as disclosed herein.
FIGS. 4A and 4B illustrate exemplary ring-back signals as received in bench top tests via a control system receiver-amplifier module without and with transmit to receive excitation signal leakage according to an embodiment disclosed herein.
FIG. 5 is an example of a sensor characterization curve.
FIG. 6 is an example of magnitude to area characterization curve.
FIG. 7 is an example of an empirically derived transmit efficacy curve.
FIG. 8 is an example of a plot of a fixed transmit frequency system frequency over time curve, where the average transmit frequency is presented with a dashed line and the average resonant frequency is presented with a solid line.
FIG. 9 is an example of a plot of a dynamically adjusted frequency system frequency over time curve, where the average transmit frequency is presented with a dashed line and the average resonant frequency is presented with a solid line.

### DETAILED DESCRIPTION

The unique physiology of the Inferior Vena Cava (IVC) presents some distinctive challenges in attempting to detect and interpret changes in its dimensions arising from changes in patient fluid state. For example, the IVC wall in a typical monitoring region (i.e., between the hepatic and renal veins) is relatively compliant compared to other vessels, which means that changes in vessel volume can result in different relative distance changes between the anterior-posterior walls as compared to the lateral-medial walls. Thus, it is quite typical that changes in fluid volume will lead to paradoxical changes in the geometry and motion of the vessel; that is, as the blood volume reduces the IVC tends to get smaller and collapse with respiration, and as the blood volume increases the IVC tends to get larger and the collapse with respiration is reduced. The present Applicant has developed new wireless sensor implants and related systems and methods in order to address these challenges and provide clinically effective wireless vascular monitors ("WVM"). In one such embodiment, the WVM comprises a resonant circuit configured as a coil implantable in the patient's vasculature ("RC-WVM'). Detailed examples of embodiments of RC-WVM systems and methods are disclosed, *inter alia,* in Applicant's U.S. Patent No. 11206992, granted on December 28, 2021, (US patent application no. 17/018,194, filed on September 11, 2020) entitled "Wireless Resonant Circuit and Variable Inductance Vascular Monitoring Implants and Anchoring Structures Therefore", q.v..

In the course of working with RC-WVM embodiments as described in the above-referenced application, Applicant has developed a number of new embodiments as disclosed herein that further improve accuracy and useability of RC-WVM implants, systems and methods as previously described. These new embodiments are described below after a basic overview discussion of one example of a RC-WVM system and its operation.

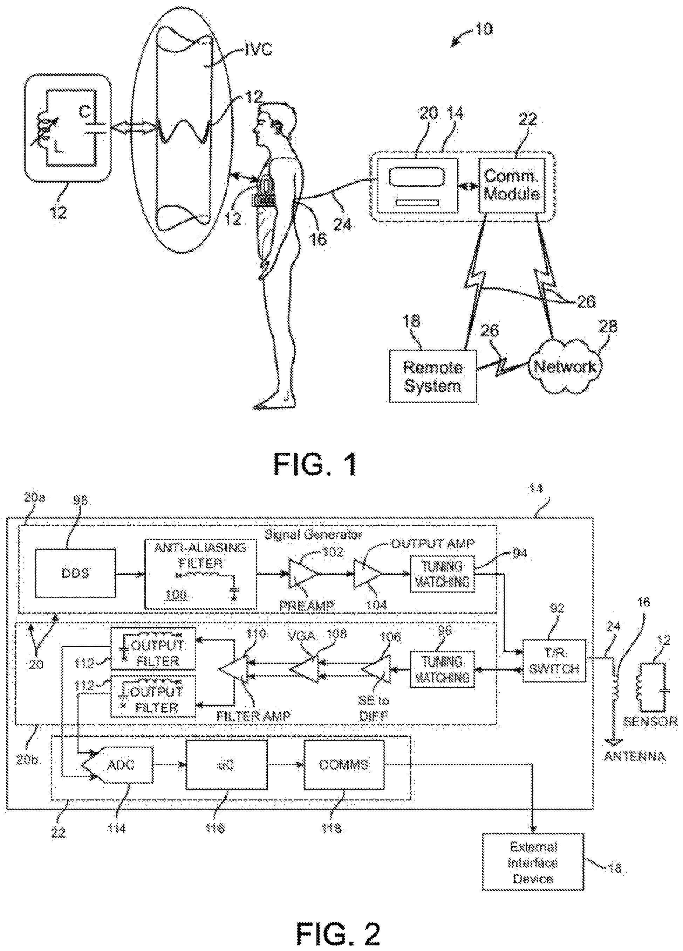

FIG. 1 provides an overview of an RC-WVM system 10 to which embodiments disclosed herein are applicable. As shown therein, such a system may generally comprise RC-WVM implant 12 configured for placement in a patient's inferior vena cava (IVC), control system 14, antenna module 16 and one or more remote systems 18 such as processing systems, user interface/displays, data storage, *etc.,* communicating with the control and communications modules through one or more data links 26. Data links 26 may be wired or remote/wireless data links. In many implementations, remote system 18 may comprise a computing device and user interface, such as a laptop, tablet or smart phone, which serves as an external interface device.

RC-WVM implants 12 generally comprise a variable inductance, constant capacitance, resonant L-C circuit formed as a collapsible and expandable coil structure, which, when positioned at a monitoring position within the patient's IVC, moves with the IVC wall as it expands and contracts due to changes in fluid volume. The variable inductance is provided by the coil structure of the implant such that the inductance changes when the dimensions of the coil (*e.g*., the area surrounded by the coil or the "sensor area") change with the IVC wall movement. The capacitive element of the circuit may be provided by a discrete capacitor or specifically designed inherent capacitance of the implant structure itself. When an excitation signal is directed at the RC-WVM implant, the resonant circuit produces a "ring-back" signal at a frequency that is characteristic of the circuit. The characteristic frequency changes based on changes in the size of the inductor, *i.e.* the coil, as it changes with the vessel wall. Because the inductance value is dependent on the geometry of the implant, which changes as mentioned above based on dimensional changes of the IVC in response to fluid state, heart rate etc., the ring-back signal can be interpreted by control system 14 to provide information as to the IVC geometry and therefore fluid state and other physiological information such as respiratory and cardiac rates.

Control system 14 comprises, for example, functional modules for signal generation, signal processing and power supply (generally comprising the excitation and feedback monitoring ("EFM") circuits and indicated as module 20, comprising signal generation module 20a and receiver-amplifier module 20b as shown in FIG. 2) and communications and data acquisition module 22 to facilitate communication and data transfer to various external or remote systems 18 through data links 26 and optionally other local or cloud-based networks 28. After analyzing signals received from RC-WVM implant 12, results may be communicated manually or automatically through an external or remote system 18 to the patient, a caregiver, a medical professional, a health insurance company, and/or any other desired and authorized parties in any suitable fashion (e.g., verbally, by printing out a report, by sending a text message or e-mail, or otherwise). As shown in FIG. 2, components of control system 14 may comprise: transmit/receive (T/R) switch 92, transmitter tuning-matching circuit 94, receiver tuning-matching circuit 96, direct digital synthesizer (DDS) 98, anti-aliasing filter 100, preamplifier 102, output amplifier 104, single ended to differential input amplifier (SE to DIFF) 106, variable gain amplifier (VGA) 108, filter amplifier (e.g., an active band-pass filter-amplifier) 110, output filters (e.g., passive, high-order low pass filters) 112, highspeed analog-to-digital converter (ADC) 114, microcontroller 116, and communications sub-module 118. Signal identification, selection and other signal processing functions subsequent to amplification and filtering may be embedded within microcontroller 116 or may be executed in an external interface device 18 such as an external computing system execution program instructions for carrying out the steps disclosed herein.

Antenna module 16 is connected to control system 14 by power and communication link 24, which may be a wired or wireless connection. Antenna module 16 creates an appropriately shaped and oriented magnetic field around RC-WVM implant 12 based on signals provided by the signal generation module 20a of control system 14 in order to excite the resonant circuit as described above. Antenna module 16 thus provides both a receive function/antenna and a transmit function/antenna. In some embodiments the transmit and receive functionality are performed by a single antenna, which is switched between transmit and receive modes, for example by transmit/receive switch 92 (which may be a single pole, double throw switch). In other embodiments, each function is performed by a separate antenna. Antenna module 16 also may optionally include an input bandpass filter to reduce noise (e.g., arising from intermodulation) and improve signal quality. The input bandpass filter may also help to improve immunity to external electromagnetic interference.

As will be appreciated by persons skilled in the art, optimal excitation of an L-C resonant circuit occurs when the excitation signal is delivered at the circuit's natural frequency. However, in an RC-WVM implant 12 as described herein, the circuit's natural frequency at any given time is unknown *a priori,* as the RC-WVM sensor size varies as per its intended use. In one embodiment, a typical sensor is qualified for patient IVC diameters nominally in the range of about 14 mm to about 28 mm. This means that overall sensor diameter range will be from somewhat less than about 14 mm to somewhat greater than 28 mm in order to detect changes in IVC dimensions above and below nominal size range. When sensor diameter lies in the lower end of that size range, e.g., below about 19 mm or even below about 15 mm, the amplitude of ring-back signal that may be produced by the sensor will be relatively low due to reduced inductive coupling and therefore can present challenges with respect to detection and accurate signal analysis. A further challenge in determining the proper excitation signal may be imposed by regulatory requirements, which typically require any such signal to have a limited bandwidth and power. These challenges can be met in a number of ways.

In one embodiment, the excitation signal provided by signal generation module 20a and delivered by antenna module 16 may be configured as a pre-defined transmit pulse (*e.g.* a single frequency burst) to energize the RC-WVM sensor. In this embodiment, the transmit pulse frequency is chosen to optimally energize the sensor on the assumption the sensor is in the lower diameter range as the smaller sensor diameter produces a lower ring-back signal amplitude. In one alternative, the transmit pulse frequency may be chosen on the assumption that the sensor is at its smallest diameter, which would have the lowest ring-back signal amplitude, thus requiring optimal excitation to ensure the ring-back signal is at a sufficiently detectable level to obtain reliable readings. The same pre-defined transmit pulse frequency is used to energize the sensor for the duration of the signal measurement, e.g., 60 seconds. However, when the vessel expands, the optimal excitation frequency changes and amplitude of the ring-back signal may decrease resulting in less reliable readings being taken.

In another embodiment, a frequency sweep function may be used to more reliably transmit the excitation signal at or close to the optimal frequency. In one example, the signal generation module 20a performs a frequency sweep function by sequentially outputting a preestablished number of transmit pulses at pre-defined frequencies over a range of expected implant natural frequencies (in one example, five transmit pulses are used). The ring-back sensor signals captured during the frequency sweep function are processed through receiver-amplifier module 20b, communications and data acquisition module 22 and optionally external devices 18. All ring-back signals (corresponding to the preestablished number of transmit pulses) are received and processed. Of the resonant frequencies detected out of the preestablished number of transmit pulses sent, the one with the highest amplitude is chosen as the optimal transmit frequency. The optimal excitation frequency is then used as the excitation transmit pulse to energize the sensor for the duration of the signal measurement, e.g., 60 seconds. Note that depending on the size of the sensor at the time of the transmit pulse sweep, all ring-back signals from the preestablished number of transmit pulses may be detected and any used as the optimal resonant frequency.

In the frequency sweep method explained above, the system selects the frequency with highest amplitude as detected during the execution of the frequency sweep function. As explained, the amplitude of the resonant frequency produced is dependent on IVC dimension (e.g., area or diameter) at the monitoring location, with larger dimensions resulting in larger signal amplitude. Employing this methodology, the system may therefore tend to choose excitation frequencies that are more optimal for larger sensor sizes. Subsequently, during signal acquisition, when the dimension of the vessel decreases (e.g. due to respiration collapse), the excitation can become suboptimal, potentially resulting in low or insufficient signal quality when the vessel collapses. Further alternative excitation frequency determination methods may be utilized to address this.

In one such further alternative embodiment, the excitation frequency is determined using a two-tier approach. Firstly, an initial excitation frequency is determined, using, for example, the frequency sweep function described above. Signal generation module 20a is therefore configured to transmit at the frequency determined by means of the frequency sweep function during an initial observation period, which should be sufficiently long to cover at least one respiration cycle. The sensor resonant frequency is assessed during this period and the highest detected frequency is subsequently chosen as the excitation frequency for the remaining of the signal measurement. This approach may favor the selection of higher frequencies, corresponding smaller sensor areas (which can be the worst case for signal quality), and as such may provide a more reliable excitation.

A limitation of the method described in the preceding paragraph is envisaged when considering a situation of significant collapse of the IVC due to respiration. In this case, as the initial frequency sweep will tend to pick a resonant frequency corresponding to larger sensor/vessel dimension, when the IVC reaches its maximum level of collapse, the resonant frequency of the sensor could deviate significantly from the excitation frequency, resulting in suboptimal excitation. This, coupled to the reduced amplitude of the sensor response (due to small sensor area) can result in unreliable resonant frequency detection (due to low signal quality) and potentially incorrect excitation frequency determination.

In order to overcome this issue, a further refinement may be employed in which the system repeatedly executes the frequency sweep function described above during a period of predefined length, which should be sufficiently long to cover at least one respiration cycle. As the excitation frequency sequentially changes between the pre-defined frequencies (including frequencies corresponding to the smallest sensor areas), a more optimal excitation is achieved in situations of large IVC collapse and small sensor. As in the method above, the system picks the highest observed resonant frequency as the excitation frequency for the remaining of the signal measurement.

In another implementation, the frequency of the excitation signal is adjusted dynamically during signal acquisition. In one embodiment, the amplitude or signal-to-noise ratio (SNR) of the response signal from the RC-WVM sensor is monitored, either continuously (for each sample) or periodically. If the signal amplitude is detected to fall below a pre-defined threshold (e.g., due to larger collapse of the IVC), a new frequency sweep (using any of the methods previously described) is executed, allowing re-tuning to the latest sensor resonant frequency.

In a further embodiment, the output frequency of signal generation module 20a is continuously adjusted after each measurement point. In this case, the resonant frequency of the sensor is computed for each acquired sample in between sample acquisitions. The excitation frequency for the next sample is therefore adjusted to the latest measured resonant frequency. Provided that the sampling rate of the system is faster than the dynamics of the IVC collapse, this method will consistently ensure optimal excitation.

Embodiments described above require signal processing algorithms for frequency detection that can be executed in real-time in communications and data acquisition module 22. Fast Fourier Transform (FFT) can be used for said purpose. However, if high resolution of the detected IVC dimension is required, the length of the required FFT could result in prohibitive computational time and would therefore be not suitable to allow frequency determination in between sample acquisitions. Alternatively, a variation of the traditional FFT such as the Zoom FFT can be used. This technique allows analyzing focusing on a given portion of the spectrum reducing this way the length of the FFT and therefore its computational time without compromising resolution of the detected frequency.

Determination of the optimal transmit frequency using any of the methods described above is a key in providing efficient excitation of the RC-WVM sensor, given that the amount of RF power that can be transmitted via antenna 16 will be subject to limits imposed by applicable regulations aimed to ensure efficient use of the frequency spectrum. As an additional means to minimize the level of intentional RF emissions, the dependency between RC-WVM sensor area and strength of the sensor response signal can be considered. As previously stated, larger sensor area will typically result in larger mutual inductance (and therefore magnetic field coupling) between the antenna 16 and the RC-WVM sensor. Taking this into account, signal generation module 20a can be controlled in such a way that the output RF power is adjusted as a function of the output frequency. In particular, maximum power is transmitted when the detected resonant frequency of the sensor is at the high end of the expected sensor bandwidth, which corresponds to the smallest sensor area and therefore weakest response. The output power is therefore monotonically reduced as the frequency decreases, facilitating thus compliance to applicable radio regulations.

In another implementation, the amplitude of the RC-WVM sensor response signal is monitored, and the output of the transmitter is dynamically adjusted, e.g. to achieve a constant signal amplitude (similar to an automatic gain control application). As described in the previous paragraph, this methodology can allow a tighter control of the emitted RF power. In addition, this methodology provides means to ensure the amplitude of the received signal does not cause saturation of the receiver stage, which can otherwise lead to inaccuracies in the signal processing algorithms that are subsequently applied in order to determine the fundamental component of the sensor.

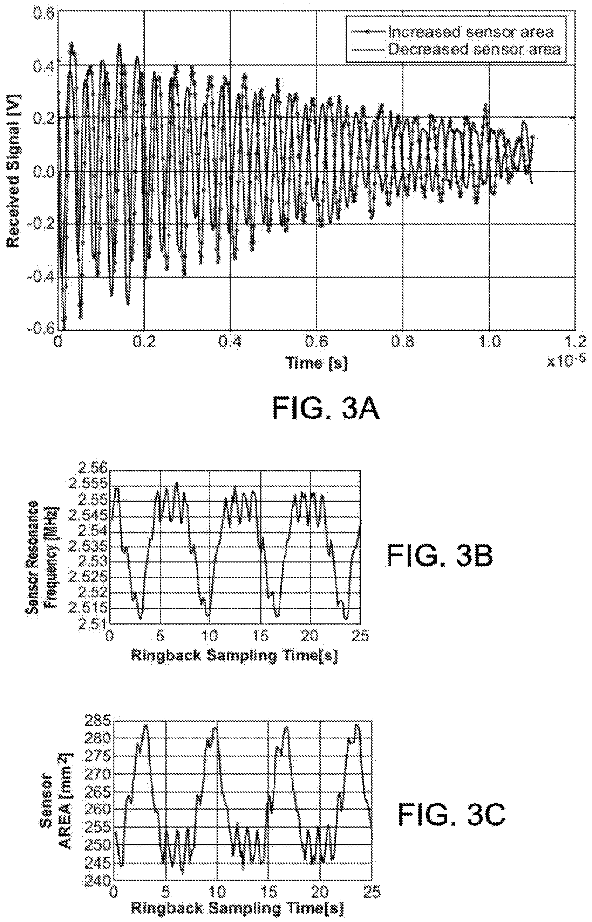

FIGS. 3A, 3B and 3C, respectively, illustrate examples of signals from *in vivo* tests, respectively, a raw ring-back signal, detection of the resonant frequency and conversion to an IVC dimension using a reference characterization curve. FIG. 3A shows the raw ring-back signal in the time domain with the resonant response of the RC-WVM implant decaying over time. Modulation of the implant geometry due to changes in IVC shape result in a change in the resonant frequency, which can be seen as the difference between the two different plotted traces. FIG. 3B shows the RC-WVM implant signal from FIG. 3A as converted into the frequency domain and plotted over time. The resonant frequency from FIG. 3A is determined (*e.g*., using fast Fourier transform) and plotted over time. The larger, slower modulation of the signal (*i.e*., the three broad peaks) indicate the respiration-induced motion of the IVC wall, while the faster, smaller modulation overlaid on this signal indicate motion of the IVC wall in response to the cardiac cycle. FIG. 3C shows the frequency modulation plotted in FIG. 3A converted to a sensor area versus time plot. (Conversion in this case was based on a characterization curve, which was determined through bench testing on a range of sample diameter lumens following standard lab/testing procedures.) FIG. 3C thus shows variations in IVC dimension at the monitoring location in response to the respiration and cardiac cycles.

As will be appreciated by persons of ordinary skill, accurate and reliable interpretation of a complex signal such as shown in FIGS. 3A-C requires good signal fidelity and confidence with respect to both the excitation signal and the ring-back signal from the RC-WVM. Embodiments disclosed herein thus provide solutions to potential problems to help ensure the best possible signal fidelity and confidence.

One way in which signal fidelity can be compromised is when defective hardware within the control system leads to inaccurate readings. A mechanism is thus needed to validate the accuracy of data produced by the system. In one embodiment, data accuracy may be validated by reading a known frequency signal created by signal generation module 20a with receiver-amplifier module 20b and confirming the output of the system matches the known input. Thus, in an embodiment a known, fixed frequency and amplitude signal portion is included within the captured signal to allow for validation of the raw data files off-line. Receiver-amplifier 20b in conjunction with the communications and data acquisition sub-module 22 starts to capture the produced signal as soon as the transmit cycle begins. The transmit signal is large in amplitude and, as such, creates a small leakage signal through the transmit/receive (T/R) switch 92 that reaches the receiver channel. Since the latter has a very large gain, the resultant signal at the receiver's output can be detected and processed in order to determine its frequency, which is known *a priori* because the transmitter has been programmed to create such a frequency. In another alternative, a known or fixed frequency signal portion may be included in the sensor raw data capture by allowing transmit/receive switch 92 to leak the known excitation signal from the transmit side to the receive side briefly when switching from transmit to receive.

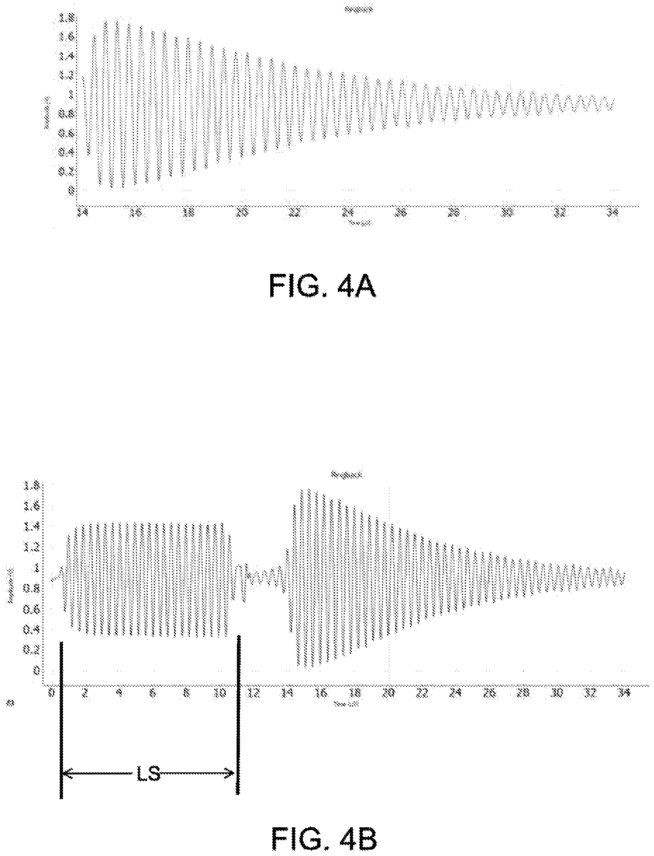

In this manner, when receiver-amplifier module 20b begins to capture the received signal, the first portion of the signal is the known frequency portion. The brief signal leakage is illustrated by comparing FIGS. 4A and 4B. FIG. 4A illustrates a ring-back signal as may be received by the control system after the RC-WVM sensor is energized by a signal from the transmit side in typical operation without any signal leakage through T/R switch 92. The signal in FIG. 4A begins at maximum amplitude at the left side when the RC-WVM coil is first energized and decays over time as energy is dissipated. Note that in this example, the ring-back signal begins at time 14 µs, which represents the time delay for the transmit signal to send and energize the sensor. (The excitation signal is delivered beginning at time 0, which is not shown in FIG. 4A, but is shown in FIG. 4B.) The signal in FIG. 4B shows the received signal when leakage through the switch is permitted as in embodiments described above. The leakage portion of the signal (LS) begins at approximately time zero because there is no delay waiting for the sensor to be energized. Then by limiting the leakage signal (LS) to a time before the sensor ring-back signal is anticipated, the leakage signal does not interfere with readings from the sensor, but at the same time provides a known frequency validation signal that can be checked against the control system output.

In one embodiment, the process of providing a leakage signal as a known frequency hardware validation signal may comprise the following:
1. An RF transmitter outputs a known pulse via an antenna to energize the sensor.
2. A transmit/receive switch is configured to allow signal leakage from the transmit side to the receive side. The receiver electronics begin to capture the receiver data while the transmitter is active.
3. The transmit/receive switch changes the antenna connection fully to the receiver electronics to detect the sensor RF response.
4. The receiver electronics continues to capture the sensor signal via an ADC.
5. The captured ADC data is stored in the microcontroller and sent to the laptop for longer-term storage. The data now includes the transmit portion of the transmit/receive cycle within the data packet. The data packet also includes the frequency programmed into the RF transmitter.
6. Data can then be validated by comparing the frequency and amplitude of the transmit portion of the data signal data against the programmed frequency and pre-defined thresholds for expected amplitude.

A further problem that can be encountered with systems of the type described herein is interference from background noise. Excessive electromagnetic noise or external electromagnetic interference from nearby devices can result in the system detecting a reading that does not relate to the sensor signal. During normal operation, the system attempts to detect a signal elicited by the sensor in response to the excitation signal that is delivered to the sensor during the transmit cycle. A sufficiently strong external signal could couple into the system and mask the sensor signal, potentially resulting in an incorrect measurement.

This problem can be solved according to the present disclosure by providing a mechanism to assess the electromagnetic background noise prior to commencement of the measurement. In one embodiment, the system is operated in normal mode, *i.e.,* the transmit mode is engaged and a known test frequency is transmitted that is sufficiently away from the expected sensor bandwidth/excitation frequency. In this way, the sensor is not energized and hence produces no ring-back signal response. The control system then toggles to receiver mode as in normal operation and any received signal is recorded. Since no response from the sensor is present (because of the "detuned" transmit frequency), the received signal is made up completely of background electromagnetic noise. Appropriate corrections or accommodations in the signal processing can then be employed based on the detected background noise. In one option, the control system assesses the power of the largest component of the background noise signal. The process is repeated a predefined number of times and an average value is obtained for more consistent measures. The computed signal level is then defined as the background noise.

A background noise evaluation process as described above is not limited to prior to commencing sensor signal recording. In other embodiments, a background noise evaluation as described can also be done at different stages or at multiple points of the sensor signal acquisition process in order to mitigate risks associated to intermittent noise sources or increased noise coupling due to patient moving, etc.

Following assessment of the background noise, the sensor signal is identified through a frequency sweep. Once the sensor response signal is detected, its amplitude is assessed and the resulting value is compared to the previously measured background noise amplitude, effectively computing the Signal to Noise Ratio (SNR). A minimum threshold level is established for the SNR. Any SNR that is below this limit indicates that the external interference is high enough to inhibit reliable measures. This can in turn alert the user to change location or remove any potential source of interference to proceed with using the system.

Use of a characterization curve to translate raw signal output of the RC-WVM sensor into physiologically relevant readings on vessel size and size changes is discussed above in connection with FIGS. 3A and 3C. In general, characterization of raw sensor signals to provide physiologically relevant readings useful to a health care provider is understood in the art. However, RC-WVM sensors as described herein can present unique characterization problems because its characteristic inductance intentionally varies by design. Further, inductance and capacitance characteristics defining the resonant circuit vary due to sensor manufacturing variability. To address these challenges in characterization of RC-WVM sensors, a number of new and different approaches may be utilized.

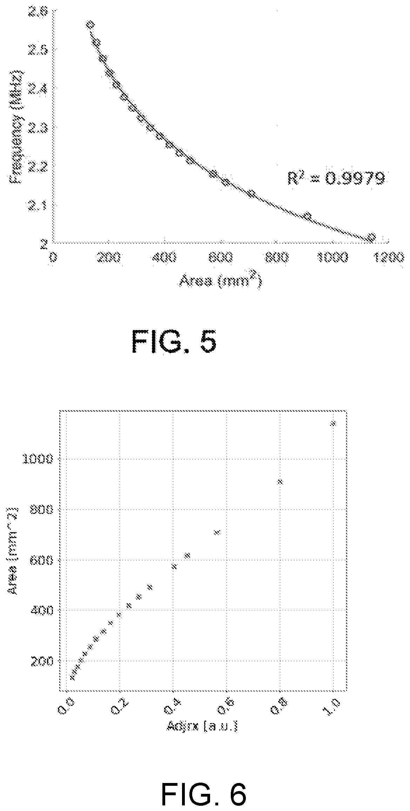

In one embodiment, a sensor characterization curve, such as shown in FIG. 5, is created by sequentially passing the RC-WVM sensor through a series of progressively larger tubes of known area and recording the corresponding frequencies. A unique curve can then be generated from these area-frequency measurements using a number of methods. For example, a curve fitting method can be employed wherein a curve is fit to the raw data by minimizing the error between the fit and the raw data. Curve fitting can be carried out using many different fit types, including, but not limited to, exponential and logarithmic fitting based on the following functions:
Logarithmic: $y = {c}_{1} . ln \left(x\right) + {c}_{2}$
Exponential: $y = {c}_{1} . {e}^{\frac{- x}{{c}_{2}}} + {c}_{3}$
In another example, interpolation may be used wherein a curve is created by interpolating between the recorded area-frequency data. A number of interpolation methods can be used, including a linear interpolation function such as:
Linear Interpolation: $y = {y}_{1} + \left(x-{x}_{1}\right) \left(\frac{{y}_{2} - {y}_{1}}{{x}_{2} - {x}_{1}}\right)$ In addition to the curve type chosen, characterization curves can be generated from individual sensor specific area-frequency data or from the average area-frequency data from a batch of sensors.

Typically, each RC-WVM sensor characterization curve is determined in a clean room during sensor manufacture. However, these curves can shift slightly after the manufacturing and sterilization process. As sensors for clinical use cannot be re-characterized post sterilization, sensor/batch specific manufacturing curves can only be created prior to sterilization. Alternatively, a reference characterization curve can also be generated from independent sensors not for clinical use post sterilization, provided they were manufactured and sterilized in a similar manner to the clinical sensors for which they will be used as a reference.

In a further embodiment, greater characterization accuracy may be achieved as follows. First, during manufacture, area versus frequency data is determined for each sensor. A characterization curve is created from this sensor or batch specific area-frequency data through curve fitting or interpolation as described above before or after sterilization. Then, a sensor measurement is taken, and the result translated into IVC dimension using the characterization curve as created in the preceding step. Measurement error arising from manufacturing variability is thus minimized through the use of sensor or batch specific characterization curves. Using a pre-determined characterization curve allows for more accurate measurements across a larger dimensional range and may avoid the need for *in vivo* calibration against imaging modalities such as intravascular ultrasound (IVUS), which present other inherent accuracy issues.

In a further alternative embodiment, information on magnitude of the response signal elicited by the resonant sensor is extracted from the sensor trace. Variation in the cross-sectional area of the inductive element of the sensor affects the amount of magnetic flux captured by the sensor, which in turn affects the magnitude of the signal that is induced in the sensor coil. The sensor response signal magnitude thus can provide additional information relating to, amongst other parameters, the cross-sectional area of the sensor, which can in cases be complementary to the information obtained from the natural frequency of the resonant circuit embedded in the sensor.

Using extracted signal magnitude information, a magnitude characterization curve can be established to map the magnitude of the received signal to the sensor cross-sectional area. As an example, this can be done by manipulating the sensor into different configurations (where the cross-sectional area is known) while determining the resultant sensor response signal magnitude as detected by the system. Such a magnitude characterization curve can be used to further characterize sensor response and accuracy of interpretation of received signals.

FIG. 6 presents an example of an area as a function of magnitude characterization curve. For a given sensor response signal, both magnitude and frequency of the resonant frequency component can be determined. Both parameters can be used jointly to determine cross-sectional area of the sensor. Using both magnitude and frequency characterization may facilitate enhanced robustness of area estimation. As an example, a situation might occur that external interference corrupts or affects the accuracy of the area estimation from sensor frequency. In this case, area detection from magnitude can be used as a secondary mechanism, that allows the detection or potentially correction of the estimated area.

While signal magnitude can be used as described above, in an *in vivo* environment for system applications described in the present disclosure detected sensor signal magnitude can be affected by other functional parameters, which may be independent of the sensor geometry and potentially may introduce error when attempting to extract sensor information from its response signal magnitude. To increase accuracy, such other functional parameters may be accounted for by techniques as described hereinafter.

Saturation of detected signal -- An implantable sensor as described in the present disclosure may be expected to operate over a relatively wide range of vessel sizes and shapes. As discussed, the magnitude of the response signal elicited by the sensor has a direct dependency with the cross-sectional area of the vessel in which it is implanted, which magnitude being lowest when the area of the vessel is at the smaller end of the scale. The receiver element in the system that is used to detect the sensor response signal shall be able to provide sufficient amplification of this worst case amplitude of the sensor response signal. However, as the area (and consequently, the sensor response signal magnitude) increases, if the same level of amplification is applied, the point will be reached eventually where linearity of the receiver amplifier cannot longer be maintained, resulting in saturation and clipping of the output signal. Said effect can affect the fidelity of the sensor signal magnitude determination, leading to potential errors in decoding the information contained in the magnitude.

In order to counteract the effect described in the preceding paragraph, the gain of the receiver amplifier can be adjusted in response to the dynamic behaviour of the implanted sensor. This can be achieved by several means, including but not limited to:
- Assessing amplitude of the sensor response signal against the dynamic range of the receiver amplifier and the Analog-to-Digital converter. If the amplitude is at the limit of the dynamic range, then the receiver gain can be reduced to bring the system back into its linear range. Such gain reduction can be achieved by using a Variable Gain Amplifier (VGA) (108 in FIG. 2), the gain of which can be modified e.g. by a control voltage that can be generated by microcontroller (116 in FIG. 2), for example by using an internal Digital-to-Analog Converter (DAC). Safety thresholds can be used to ensure gain is adjusted before saturation occurs (e.g. when the detected signal amplitude is rising and approaching the upper limit of the dynamic range, a gain reduction is triggered when the signal amplitude reaches a predetermined threshold. These thresholds can be selected as a trade-off between likelihood of saturation and effective dynamic range of the receiver stage (e.g. use of low thresholds would reduce the probability of saturation, in particular due to fast changing signal but would result in the use of very limited dynamic range, therefore reducing the effective resolution of the Analog-to-Digital converting stage. As an example, for a 12 bits ADC (where the maximum number of ADC counts is 4095), a receiver gain control function can be implemented such that when the peak value of the amplified response signal from the RC-WVM sensor reaches a count corresponding to 75% of the maximum ADC count (i.e., 3064), the receiver gain is reduced.
- A second lower threshold can be added to the method described above, so that when the sensor signal amplitude falls below that threshold, the gain is increased, so that a larger portion of the receiver amplifier dynamic range is occupied, ensuring this way optimal use of the resolution of the Analog-to-Digital converter. As an example, for a 12 bits ADC (where the maximum number of ADC counts is 4095), a receiver gain control function can be implemented such that when the peak value of the amplified response signal from the RC-WVM sensor is below the count corresponding to 25% of the maximum ADC count (i.e., 1024), the receiver gain is increased.
- The optimal receiver gain can be computed from the sensor resonant frequency. In particular, an inverse correlation exists between cross-sectional areas of the sensor and its resonant frequency, with lower frequencies corresponding to larger areas, which in turn results in larger signal magnitude as previously discussed. On this basis, it is possible to create a mapping of frequency to gain that would allow adjustment of the receiver gain in response to the detected sensor resonant frequency.

Excitation signal efficacy - Another item affecting the overall signal magnitude is the efficacy of the excitation signal, which is defined as the amount of energy of the excitation signal transmitted by the control system that is effectively captured by resonant circuit of the RC-WVM sensor. As described above in paragraph 0016, maximum energy transfer to the sensor resonant circuit occurs when the frequency of the excitation signal equates the resonant frequency of the sensor. Any disparity between both will result in reduced efficacy of the excitation signal, resulting in reduced magnitude of the response signal elicited by the RC-WVM sensor. This could result in inaccurate area estimation (e.g., the sensor area might be assumed to be smaller than it actually is due to the reduced magnitude, which in reality is the result of inefficient sensor excitation). In order to mitigate this issue, a transmit efficacy function can be derived, which takes the form of a transmit efficiency coefficient factor (which would be a number between 0 and 1), and said coefficient being a function of the difference between transmit and receive signal frequencies (with the coefficient being equal to 1 when both transmit and receive signal frequencies are identical). By knowing the transmit efficacy, it is possible to account for the effect of the sub-optimal RC-WVM sensor excitation on the resulting signal magnitude and therefore eliminate or reduce this confounding factor, which ultimately results in improved reliability of the magnitude of the response signal elicited by the RC-WVM sensor as a predictor of the sensor cross-sectional area.

In one embodiment, the effect of sub-optimal sensor excitation can be accounted for by adjusting the measured signal magnitude upward by the inverse of the transmit efficacy function. This can be done by multiplying the measured signal magnitude by the inverse of the efficacy function (in other words, dividing by the efficacy function to give the adjusted signal magnitude). The sensed parameter, such as lumen area, is then determined with the magnitude characterization curve using the adjusted signal magnitude instead of the measured signal magnitude. For example, if the measured signal magnitude is 70 a.u. and the efficacy function is 0.7, then the adjusted signal magnitude is 100 a.u. (*i.e*., 70 a.u. / 0.7).

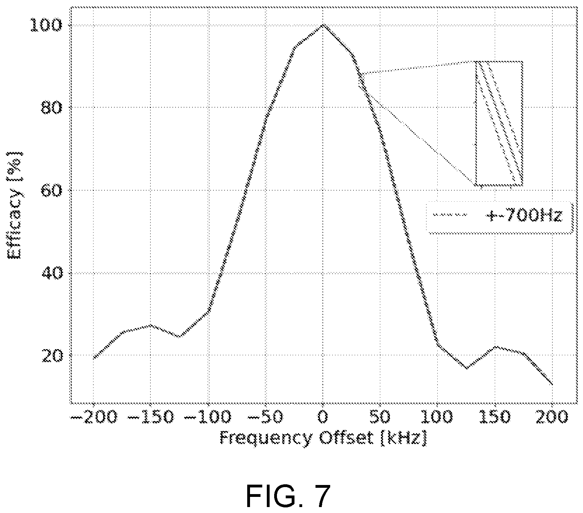

This transmit efficacy function can be derived theoretically *e.g*., by computing the spectrum of the transmit signal and the frequency response curve of the sensor and by shifting the transmit signal frequency over the expected range of transmission. In a possible implementation, the spectrum of the transmit signal is computed (e.g. using Fourier Transform). The magnitude of this spectrum is then sampled at the resonant frequency of the RC-WVM sensor. For a transmit signal consisting of a pre-defined number of pulses of sine wave for which the spectrum will correspond to a Sin function, the following equation can be used for calculating the efficacy from the RC-WVM sensor resonant frequency (*f_rx*) and transmit (*f_tx*) frequency: $\left|\frac{sin \left(π∗\left({f}_{rx}-{f}_{tx}\right)∗τ\right)}{π ∗ \left({f}_{rx}-{f}_{tx}\right) ∗ τ}\right|$ Alternatively, the transmit efficacy function can be derived empirically, by acquiring a sensor signal for a given area, starting with optimal alignment between transmit and received frequency and subsequently shifting the transmit frequency both upwards and downwards while assessing the effect on the magnitude of the sensor response signal, as illustrated, for example by FIG. 7, which presents an example of an empirically derived transmit efficacy curve.

Dynamic transmit signal frequency adjustment based on signal efficacy -- An alternative approach for transmit signal frequency determination comprises dynamic frequency adjustment based on a difference between a detected sensor resonant frequency and energizing signal transmit frequency. In this technique, the system performs an initial frequency sweep transmitting at a set of discrete frequencies over the expected range of sensor resonant frequencies. An initial transmit frequency is derived using this approach, as previously disclosed. The frequency of the sensor response signal is subsequently monitored in each acquisition and the difference between the detected frequency and the transmit frequency is computed. A threshold can therefore be established such that the transmit frequency will remain unchanged as long as the computed difference between the frequency of the transmitted and received signals is below the established threshold value. If the difference in frequency between transmitted and received signals crosses the established threshold, the transmit frequency is re-adjusted to match the last detected sensor resonant frequency.

To implement dynamic transmit signal frequency adjustment, the signal generation circuit should be able to create a signal of variable frequency, which is described above in connection with frequency sweep (see paragraph 0018). In one example implementation based on system 14 as exemplified in the FIG. 2 block diagram, there is a feedback loop that allows the system to detect the sensor resonant frequency (*e.g*. by means of Zoom FFT - as discussed in paragraph 0025) and reconfigure the frequency of the DDS in the transmitter circuit to match the detected sensor frequency.

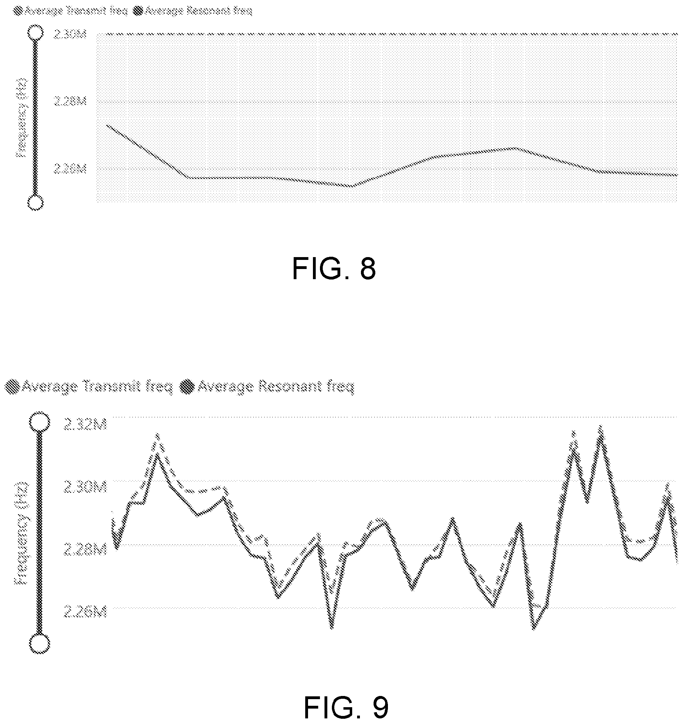

FIGS. 8 and 9 illustrate, respectively, an example where the transmit frequency is fixed and another example where the transmit frequency is dynamically adjusted to approximate the resonant frequency of the RC-WVM sensor. By adjusting the transmit frequency, the system will tend to operate close to the maximum transmit efficacy (as defined above), resulting in maximum signal magnitude which in turn results in improved reliability of the magnitude of the response signal elicited by the RC-WVM sensor as a predictor of the sensor cross-sectional area.

The threshold for transmit signal frequency update can be defined as a trade-off between the bandwidth of the transmit excitation signal and the expected range for sensor resonant frequency change resulting from collapse of the IVC *e.g.,* due to respiration. In one example, such a predetermined threshold may be set as a specific value for the difference between the sensor resonant frequency and the excitation signal transmit frequency, such as a difference of about 25 kHz or greater. In another example, the predetermined threshold may be set based on a minimum transmit efficacy function, such as a transmit efficacy function of 0.7 or less. Following this approach, optimal or near optimal excitation can be achieved while minimizing the number of transmit frequency changes and maintaining quasi-fixed frequency operation.

The foregoing has been a detailed description of illustrative embodiments of the invention. It is noted that in the present specification and claims appended hereto, conjunctive language such as is used in the phrases "at least one of X, Y and Z" and "one or more of X, Y, and Z," unless specifically stated or indicated otherwise, shall be taken to mean that each item in the conjunctive list can be present in any number exclusive of every other item in the list or in any number in combination with any or all other item(s) in the conjunctive list, each of which may also be present in any number. Applying this general rule, the conjunctive phrases in the foregoing examples in which the conjunctive list consists of X, Y, and Z shall each encompass: one or more of X; one or more of Y; one or more of Z; one or more of X and one or more of Y; one or more of Y and one or more of Z; one or more of X and one or more of Z; and one or more of X, one or more of Y and one or more of Z.

Various modifications and additions can be made without departing from the and scope of this invention. Features of each of the various embodiments described above may be combined with features of other described embodiments as appropriate in order to provide a multiplicity of feature combinations in associated new embodiments. Furthermore, while the foregoing describes a number of separate embodiments, what has been described herein is merely illustrative of the application of the principles of the present invention. Additionally, although particular methods herein may be illustrated and/or described as being performed in a specific order, the ordering is highly variable within ordinary skill to achieve aspects of the present disclosure. Accordingly, this description is meant to be taken only by way of example, and not to otherwise limit the scope of this invention.

Exemplary embodiments have been disclosed above and illustrated in the accompanying drawings. It will be understood by those skilled in the art that various changes, omissions and additions may be made to that which is specifically disclosed herein without departing from the scope of the present invention.

## Claims

1. A method for controlling a wireless resonant circuit sensor, the sensor including a variable inductance coil that changes resonant frequency in response to a change in a monitored physical parameter and produces a ring-back signal with a signal magnitude correlated to the physical parameter when energized, the method comprising:
outputting an excitation signal selected to produce the ring-back signal from said sensor;
receiving the ring-back signals from said sensor at a receiving amplifier;
comparing the magnitude of the sensor ring-back signal to a dynamic range of the receiving amplifier; and
reducing receiving amplifier gain when compared magnitude is at or exceeds a magnitude dynamic range of the receiving amplifier.

2. The method of claim 1, wherein said reducing receiving amplifier gain comprises reducing said gain to be within a linear range of the receiving amplifier; or reducing said gain when ring-back signal magnitude is increasing and reaches a predetermined magnitude threshold.

3. The method of any of claims 1-2, wherein:
the receiving amplifier comprises an amplifier circuit including an analog-to-digital converter, ADC; and
a receiver gain control function is implemented to reduce receiving amplifier gain when a peak value of the amplified response signal from said sensor reaches a count corresponding to a predetermined percentage of a maximum ADC count.

4. The method of any of claims 1-3, further comprising adjusting receiver amplifier gain in response to a detected sensor resonant frequency based on a predetermined map of sensor frequency to gain.

5. The method of any of claims 1-4, further comprising:
adjusting the received ring-back signal magnitude by a transmit efficacy function to provide an adjusted ring-back signal magnitude;
comparing the adjusted ring-back signal magnitude to a physical parameter - magnitude correlation to determine a value for the physical parameter based on said correlation.

6. The method of claim 5, wherein the transmit efficacy function is calculated as equal to $\left|\frac{sin \left(π∗\left({f}_{rx}-{f}_{tx}\right)∗τ\right)}{π ∗ \left({f}_{rx}-{f}_{tx}\right) ∗ τ}\right|$ where fᵣₓ is the sensor resonant frequency and fₜₓ is the energizing signal transmit frequency.

7. The method of claim 5, wherein the transmit efficacy function determined based on an empirically derived transmit frequency efficacy curve.

8. The method of any of claims 1-7, further comprising:
determining physical parameter versus magnitude data for at least one said sensor prior to placement in a patient;
creating a magnitude versus physical parameter characterization curve for the at least one sensor based on said physical parameter versus magnitude data through curve fitting or interpolation;
taking a measurement with the sensor; and
translating the sensor measurement into a value for the physical parameter using said characterization curve.

9. The method of claim 8, further comprising minimizing physical parameter measurement error arising from sensor manufacturing variability through use of sensor or sensor batch specific characterization curves.

10. A method for characterizing a resonant circuit sensor to correlate sensor output to a measured physical parameter, wherein said sensor comprises a variable inductance coil that changes resonant frequency in response to a change in the physical parameter by producing, when energized, a ring-back signal having a signal magnitude correlateable to the physical parameter, the method comprising:
determining physical parameter value versus signal magnitude data over a range of parameter values and signal magnitudes for at least one said sensor prior to placement in a patient; and
creating a signal magnitude characterization curve for the at least one sensor by plotting a curve with said signal magnitude data using curve fitting or interpolation techniques.

11. The method of claim 10, wherein the physical parameter is an internal vascular lumen dimension comprising area of the lumen, said sensor being implantable within a vascular lumen and expandable and contractable therewith, wherein t said determining comprises sequentially placing the sensor in a series of progressively larger or smaller tubes of known dimension and recording the corresponding ring-back signal magnitudes when energized in each different-sized tube.

12. The method of claim 11, further comprises:
during manufacture, determining a vascular dimension versus signal magnitude data set for each sensor in a sensor batch; and
creating the characterization curve from the senor batch dimension-magnitude data through curve fitting or interpolation prior to sterilization of the sensors.

13. The method of any of claims 10-12, wherein the ring-back signal further includes a frequency correlateable to the physical parameter, and said method further comprises:
determining physical parameter value versus frequency data over a range of parameter values and frequencies for said at least one said sensor prior to placement in a patient;
creating a frequency characterization curve for the at least one sensor by plotting a curve with said frequency data using curve fitting or interpolation techniques; and
correlating sensor output for the at least one said sensor with the measured parameter based on both signal magnitude characterization and frequency characterization.

14. A method for controlling a wireless resonant circuit sensor, the sensor including a variable inductance coil that changes resonant frequency in response to a change in a monitored physical parameter and produces a ring-back signal at a frequency or magnitude correlated to the physical parameter when energized, the method comprising:
outputting a sensor energizing signal at an initial transmit frequency;
receiving the ring-back signal at a ring-back frequency from the sensor in response to the sensor energizing signal;
determining a difference between the transmit frequency and the ring-back frequency;
periodically repeating said outputting, receiving and determining while said difference between the transmit frequency and ring-back frequency is below a predetermined threshold;
changing the sensor energizing signal transmit frequency to a new transmit frequency matching the ring-back frequency of a last received ring-back signal when said difference meets or exceeds the predetermined threshold; and
periodically repeating said outputting at the new transmit frequency and
thereafter repeating said receiving and determining.

15. The method of claim 14, wherein said outputting a sensor energizing signal at an initial transmit frequency comprises:
outputting at least one sensor energizing signal frequency sweep comprising a preestablished number of transmit pulses at pre-defined frequencies over a range of expected sensor resonant frequencies;
receiving the ring-back signals for each of the sequentially output transmit pulses;
transmitting at least one initial transmit pulse for a predetermined initial period, wherein the at least one initial transmit pulse comprises one of - a pulse frequency corresponding to the highest amplitude ring-back signal received from the at least one frequency sweep; or plural said energizing signal frequency sweeps;
receiving plural test ring-back signals in response to at least one initial transmit pulse transmitted over the initial period;
identifying an initial ring-back signal corresponding to a preferred energizing signal pulse frequency;
selecting said preferred energizing signal pulse frequency as a measurement transmit pulse frequency; and
outputting said sensor energizing signal at the initial frequency as measurement transmit pulses at the measurement transmit pulse frequency for a subsequent measurement period.

## Patentansprüche

1. Ein Verfahren zum Steuern eines drahtlosen Schwingkreissensors, wobei der Sensor eine Spule mit variabler Induktivität aufweist, die die Resonanzfrequenz in Reaktion auf eine Änderung eines überwachten physikalischen Parameters ändert und wenn angeregt ein Ring-Back-Signal mit einer mit dem physikalischen Parameter korrelierten Signalmagnitude erzeugt, wobei das Verfahren Folgendes aufweist:
Ausgeben eines Anregungssignals, das zum Erzeugen des Ring-Back-Signals von dem besagten Sensor ausgewählt ist;
Empfangen der Ring-Back-Signale von dem besagten Sensor an einem Empfangsverstärker;
Vergleichen der Ring-Back-Signalmagnitude des Sensors mit einem dynamischen Bereich des Empfangsverstärkers; und
Reduzieren der Empfangsverstärkung, wenn die verglichene Magnitude einem dynamischen Magnitudenbereich des Empfangsverstärkers entspricht oder diesen überschreitet.

2. Das Verfahren nach Anspruch 1, wobei das besagte Reduzieren der Empfangsverstärkung Folgendes aufweist:
Reduzieren der besagten Verstärkung, um innerhalb eines linearen Bereichs des Empfangsverstärkers zu liegen; oder
Reduzieren der besagten Verstärkung, wenn die Ring-Back-Signalmagnitude zunimmt und einen vorbestimmten Magnitude-Schwellenwert erreicht.

3. Das Verfahren nach einem der Ansprüche 1 bis 2, wobei:
der Empfangsverstärker eine Verstärkerschaltung aufweist, die einen Analog-Digital-Wandler, ADC, aufweist; und
eine Empfangsverstärkungssteuerungsfunktion implementiert ist, um die Empfangsverstärkung zu reduzieren, wenn ein Spitzenwert des verstärkten Antwortsignals von dem besagten Sensor einen Zählwert erreicht, der einem vorbestimmten Prozentsatz eines maximalen ADC-Zählwerts entspricht.

4. Das Verfahren nach einem der Ansprüche 1 bis 3, ferner aufweisend das Anpassen der Empfangsverstärkung in Reaktion auf eine detektierte Sensorresonanzfrequenz basierend auf einer vorbestimmten Sensorfrequenz-Verstärkungszuordnung.

5. Das Verfahren nach einem der Ansprüche 1 bis 4, ferner Folgendes aufweisend:
Anpassen der empfangenen Ring-Back-Signalmagnitude durch eine Sendeeffizienzfunktion, um eine angepasste Ring-Back-Signalmagnitude bereitzustellen;
Vergleichen der angepassten Ring-Back-Signalmagnitude mit einer Parameter-Magnitude-Korrelation,
um einen Wert für den physikalischen Parameter basierend auf der besagten Korrelation zu bestimmen.

6. Das Verfahren nach Anspruch 5, wobei die Sendeeffizienzfunktion berechnet wird als gleich zu $\left|\frac{sin \left(π∗\left({f}_{rx}-{f}_{tx}\right)∗τ\right)}{π ∗ \left({f}_{rx}-{f}_{tx}\right) ∗ τ}\right|$ wobei *fᵣₓ* die Sensorresonanzfrequenz und *fₜₓ* die Erregersignalsendefrequenz ist.

7. Das Verfahren nach Anspruch 5, wobei die Sendeeffizienzfunktion basierend auf einer empirisch abgeleiteten Sendefrequenzeffizienzkurve bestimmt wird.

8. Das Verfahren nach einem der Ansprüche 1 bis 7, ferner Folgendes aufweisend:
Bestimmen von physikalischer Parameter-versus-Magnitude-Daten für wenigstens einen besagten Sensor vor der Platzierung in einem Patienten;
Erstellen einer Charakterisierungskurve für Magnitude-versusphysikalischer Parameter für den wenigstens einen Sensor basierend auf den besagten Parameter-Magnitude-Daten durch Kurvenanpassung oder Interpolation;
Durchführen einer Messung mit dem Sensor; und
Umwandeln der Sensormessung in einen Wert für den physikalischen Parameter unter Verwendung der besagten Charakterisierungskurve.

9. Das Verfahren nach Anspruch 8, ferner aufweisend das Minimieren von Messfehlern des physikalischen Parameters, die aus Fertigungsstreuung des Sensors entstehen, durch Verwendung sensorspezifischer oder chargenspezifischer Charakterisierungskurven.

10. Ein Verfahren zum Charakterisieren eines Schwingkreissensors, um den Sensorausgang mit einem gemessenen physikalischen Parameter zu korrelieren, wobei der besagte Sensor eine Spule mit variabler Induktivität aufweist, die die Resonanzfrequenz in Reaktion auf eine Änderung des physikalischen Parameters ändert, indem sie, wenn angeregt, ein Ring-Back-Signal mit einer mit dem physikalischen Parameter korrelierbaren Signalmagnitude erzeugt, wobei das Verfahren Folgendes aufweist:
Bestimmen von physikalischer Parameterwert-versus-Signalmagnitude-Daten über einen Bereich von Parameterwerten und Signalmagnituden für wenigstens einen besagten Sensor vor der Platzierung in einem Patienten; und
Erstellen einer Signalmagnitude-Charakterisierungskurve für den wenigstens einen Sensor durch Aufzeichnen einer Kurve mit den besagten Signalmagnitude-Daten unter Verwendung von Kurvenanpassungs- oder Interpolationstechniken.

11. Das Verfahren nach Anspruch 10, wobei der physikalische Parameter eine innere Gefäßlumenabmessung ist, die die Fläche des Lumens aufweist, wobei der besagte Sensor innerhalb eines Gefäßlumens implantierbar und damit ausdehnbar und zusammenziehbar ist, wobei *t* das besagte Bestimmen das sequenzielle Platzieren des Sensors in einer Reihe von progressiv größeren oder kleineren Röhren mit bekannter Abmessung und das Aufzeichnen der entsprechenden Ring-Back-Signalmagnituden bei Anregung in jeder Röhre unterschiedlicher Größe aufweist.

12. Das Verfahren nach Anspruch 11, ferner aufweisend:
während der Herstellung, Bestimmen eines Gefäßabmessungs-versus-Signalmagnitude-Datensatzes für jeden Sensor in einer Sensorcharge; und
Erstellen der Charakterisierungskurve aus den Sensorchargen-Abmessungsmagnitude-Daten durch Kurvenanpassung oder Interpolation vor der Sterilisation der Sensoren.

13. Das Verfahren nach einem der Ansprüche 10 bis 12, wobei das Ring-Back-Signal ferner eine mit dem physikalischen Parameter korrelierbare Frequenz aufweist und das besagte Verfahren ferner Folgendes aufweist:
Bestimmen von physikalischer Parameter-versus-Frequenzdaten über einen Bereich von Parameterwerten und Frequenzen für den besagten wenigstens einen besagten Sensor vor der Platzierung in einem Patienten;
Erstellen einer Frequenzcharakterisierungskurve für den wenigstens einen Sensor durch Aufzeichnen einer Kurve mit den besagten Frequenzdaten unter Verwendung von Kurvenanpassungs- oder Interpolationstechniken; und
Korrelieren des Sensorausgangs für den wenigstens einen besagten Sensor mit dem gemessenen Parameter basierend auf sowohl der Signalmagnitude-Charakterisierung als auch der Frequenzcharakterisierung.

14. Ein Verfahren zum Steuern eines drahtlosen Schwingkreissensors, wobei der Sensor eine Spule mit variabler Induktivität aufweist, die die Resonanzfrequenz in Reaktion auf eine Änderung eines überwachten physikalischen Parameters ändert und wenn angeregt ein Ring-Back-Signal bei einer mit dem physikalischen Parameter korrelierten Frequenz oder Magnitude erzeugt, wobei das Verfahren Folgendes aufweist:
Ausgeben eines Sensorerregersignals bei einer Anfangssendefrequenz;
Empfangen des Ring-Back-Signals bei einer Ring-Back-Frequenz von dem Sensor in Reaktion auf das Sensorerregersignal;
Bestimmen einer Differenz zwischen der Sendefrequenz und der Ring-Back-Frequenz;
periodisches Wiederholen des besagten Ausgebens, Empfangens und Bestimmens, während die besagte Differenz zwischen der Sendefrequenz und der Ring-Back-Frequenz unterhalb eines vorbestimmten Schwellenwerts liegt;
Ändern der Sendefrequenz des Sensorerregersignals auf eine neue Sendefrequenz, die der Ring-Back-Frequenz eines zuletzt empfangenen Ring-Back-Signals entspricht, wenn die besagte Differenz den vorbestimmten Schwellenwert erreicht oder überschreitet; und
periodisches Wiederholen des besagten Ausgebens bei der neuen Sendefrequenz und anschließendes Wiederholen des besagten Empfangens und Bestimmens.

15. Das Verfahren nach Anspruch 14, wobei das besagte Ausgeben eines Sensorerregersignals bei einer Anfangssendefrequenz Folgendes aufweist:
Ausgeben wenigstens eines Erregersignalfrequenzdurchlaufs, der eine vorgegebene Anzahl von Sendeimpulsen bei vordefinierten Frequenzen über einen Bereich erwarteter Sensorresonanzfrequenzen aufweist;
Empfangen der Ring-Back-Signale für jeden der sequenziell ausgegebenen Sendeimpulse;
Übertragen wenigstens eines Anfangssendeimpulses für einen vorbestimmten Anfangszeitraum, wobei der wenigstens eine Anfangssendeimpuls eines der Folgenden aufweist -
eine Impulsfrequenz, die dem Ring-Back-Signal mit der höchsten Amplitude entspricht, das von dem wenigstens einen Erregersignalfrequenzdurchlauf empfangen wurde; oder
mehrere der besagten Erregersignalfrequenzdurchläufe;
Empfangen mehrerer Test-Ring-Back-Signale in Reaktion auf wenigstens einen über den Anfangszeitraum übertragenen Anfangssendeimpuls;
Identifizieren eines anfänglichen Ring-Back-Signals, das einer bevorzugten Erregersignalimpulsfrequenz entspricht;
Auswählen der besagten bevorzugten Erregersignalimpulsfrequenz als Messsendeimpulsfrequenz; und
Ausgeben des besagten Sensorerregersignals bei der Anfangsfrequenz als Messsendeimpulse bei der Messsendeimpulsfrequenz für einen nachfolgenden Messzeitraum.

## Revendications

1. Procédé de contrôle d'un capteur à circuit résonant sans fil, le capteur comprenant une bobine à inductance variable qui modifie la fréquence de résonance en réponse à une variation d'un paramètre physique surveillé et produit un signal de retour ayant une amplitude du signal corrélée au paramètre physique lorsqu'il est excité, le procédé comprenant :
émettre un signal d'excitation sélectionné pour produire le signal de retour à partir dudit capteur ;
recevoir des signaux de retour provenant dudit capteur à un amplificateur de réception ;
comparer l'amplitude du signal de retour du capteur à une plage dynamique de l'amplificateur de réception ; et
réduire le gain de l'amplificateur de réception lorsque l'amplitude comparée est égale ou supérieure à une plage dynamique d'amplitude de l'amplificateur de réception.

2. Procédé selon la revendication 1, dans lequel la réduction du gain de l'amplificateur de réception comprend la réduction dudit gain pour qu'il soit dans une plage linéaire de l'amplificateur de réception ; ou la réduction dudit gain lorsque l'amplitude du signal de retour augmente et atteint un seuil d'amplitude prédéterminé.

3. Procédé selon l'une quelconque des revendications 1 à 2, dans lequel :
l'amplificateur de réception comprend un circuit amplificateur comprenant un convertisseur analogique-numérique (CAN) ; et
une fonction de contrôle du gain du récepteur est mise en œuvre pour réduire le gain de l'amplificateur de réception lorsqu'une valeur de crête du signal de réponse amplifié provenant dudit capteur atteint une valeur correspondant à un pourcentage prédéterminé de la valeur maximale du CAN.

4. Procédé selon l'une quelconque des revendications 1 à 3, comprenant en outre l'ajustement du gain de l'amplificateur de réception en réponse à une fréquence de résonance du capteur détectée sur la base d'une cartographie prédéterminée de la fréquence du capteur au gain.

5. Procédé selon l'une quelconque des revendications 1 à 4, comprenant en outre :
ajuster l'amplitude du signal de retour reçu par une fonction d'efficacité de transmission afin de fournir une amplitude ajustée du signal de retour ;
comparer l'amplitude ajustée du signal de retour à une corrélation paramètre physique - amplitude afin de déterminer une valeur pour le paramètre physique sur la base de ladite corrélation.

6. Procédé selon la revendication 5, dans lequel la fonction d'efficacité de transmission est calculée comme étant égale à $\left|\frac{sin \left(π∗\left({f}_{rx}-{f}_{tx}\right)∗τ\right)}{π ∗ \left({f}_{rx}-{f}_{tx}\right) ∗ T}\right|$ où fᵣₓ est la fréquence de résonance du capteur et fₜₓ est la fréquence d'émission du signal d'excitation.

7. Procédé selon la revendication 5, dans lequel la fonction d'efficacité de transmission est déterminée sur la base d'une courbe d'efficacité de fréquence de transmission dérivée empiriquement.

8. Procédé selon l'une quelconque des revendications 1 à 7, comprenant en outre :
déterminer des données paramètre physique versus amplitude pour au moins un desdits capteurs avant la mise en place chez un patient ;
créer une courbe de caractérisation amplitude versus paramètre physique pour ledit au moins un capteur sur la base desdites données paramètre physique versus amplitude par ajustement de courbe ou interpolation ;
réaliser une mesure avec le capteur ; et
traduire la mesure du capteur en une valeur pour le paramètre physique à l'aide de ladite courbe de caractérisation.

9. Procédé selon la revendication 8, comprenant en outre la minimisation de l'erreur de mesure du paramètre physique résultant de la variabilité de fabrication du capteur par l'utilisation de courbes de caractérisation spécifiques au capteur ou au lot de capteurs.

10. Procédé de caractérisation d'un capteur à circuit résonant afin de corréler la sortie du capteur à un paramètre physique mesuré, dans lequel le capteur comprend une bobine à inductance variable qui modifie la fréquence de résonance en réponse à une variation du paramètre physique en produisant, lorsqu'il est excité, un signal de retour ayant une amplitude du signal corrélable au paramètre physique, le procédé comprenant :
déterminer des données de valeur du paramètre physique versus amplitude du signal sur une plage de valeurs de paramètre et d'amplitudes de signal pour au moins un desdits capteurs avant la mise en place chez un patient ; et
créer une courbe de caractérisation de l'amplitude du signal pour ledit au moins un capteur en traçant une courbe avec lesdites données d'amplitude du signal par des techniques d'ajustement de courbe ou d'interpolation.

11. Procédé selon la revendication 10, dans lequel le paramètre physique est une dimension interne de la lumière vasculaire comprenant la surface de la lumière, ledit capteur étant implantable à l'intérieur d'une lumière vasculaire et expansible et contractile avec celle-ci, dans lequel ladite détermination comprend le placement séquentiel du capteur dans une série de tubes de dimension connue, de plus en plus grands ou plus petits, et l'enregistrement des amplitudes correspondantes du signal de retour lorsqu'il est excité dans chaque tube de taille différente.

12. Procédé selon la revendication 11, comprenant en outre :
lors de la fabrication, déterminer un ensemble de données dimension vasculaire versus amplitude du signal pour chaque capteur dans un lot de capteurs ; et
créer la courbe de caractérisation à partir des données dimension-amplitude du lot de capteurs par ajustement de courbe ou interpolation avant la stérilisation des capteurs.

13. Procédé selon l'une quelconque des revendications 10 à 12, dans lequel le signal de retour comprend en outre une fréquence corrélable au paramètre physique, et ledit procédé comprend en outre :
déterminer des données de valeur du paramètre physique versus fréquence sur une plage de valeurs de paramètre et de fréquences pour ledit au moins un capteur avant la mise en place chez un patient ;
créer une courbe de caractérisation en fréquence pour ledit au moins un capteur en traçant une courbe avec lesdites données de fréquence par des techniques d'ajustement de courbe ou d'interpolation ; et
corréler la sortie du capteur pour ledit au moins un capteur avec le paramètre mesuré sur la base à la fois de la caractérisation de l'amplitude du signal et de la caractérisation en fréquence.

14. Procédé de contrôle d'un capteur à circuit résonant sans fil, le capteur comprenant une bobine à inductance variable qui modifie la fréquence de résonance en réponse à une variation d'un paramètre physique surveillé et produit un signal de retour à une fréquence ou une amplitude corrélée au paramètre physique lorsqu'il est excité, le procédé comprenant :
émettre un signal d'excitation du capteur à une fréquence d'émission initiale ; la réception du signal de retour à une fréquence de retour provenant du capteur en réponse au signal d'excitation du capteur ;
déterminer une différence entre la fréquence d'émission et la fréquence de retour ;
répéter périodiquement lesdites étapes d'émission, de réception et de détermination tant que ladite différence entre la fréquence d'émission et la fréquence de retour est inférieure à un seuil prédéterminé ;
modifier la fréquence d'émission du signal d'excitation du capteur à une nouvelle fréquence d'émission correspondant à la fréquence de retour du dernier signal de retour reçu lorsque ladite différence atteint ou dépasse le seuil prédéterminé ; et
répéter périodiquement ladite émission à la nouvelle fréquence d'émission et, par la suite, répéter lesdites étapes de réception et de détermination.

15. Procédé selon la revendication 14, dans lequel ladite émission d'un signal d'excitation du capteur à une fréquence d'émission initiale comprend :
émettre au moins un balayage en fréquence du signal d'excitation du capteur comprenant un nombre préétabli d'impulsions d'émission à des fréquences prédéfinies sur une plage de fréquences de résonance attendues du capteur ;
recevoir des signaux de retour pour chacune des impulsions d'émission séquentiellement émises ;
émettre au moins une impulsion d'émission initiale pour une période initiale prédéterminée, dans laquelle ladite au moins une impulsion d'émission initiale comprend l'une des options suivantes -
une fréquence d'impulsion correspondant au signal de retour d'amplitude la plus élevée reçu à partir du ou desdits balayages en fréquence ; ou
plusieurs dits balayages en fréquence du signal d'excitation ;
recevoir plusieurs signaux de retour de test en réponse à au moins une impulsion d'émission initiale émise pendant la période initiale ;
identifier un signal de retour initial correspondant à une fréquence d'impulsion d'excitation préférée ;
sélectionner ladite fréquence d'impulsion d'excitation préférée comme fréquence d'impulsion d'émission de mesure ; et
émettre ledit signal d'excitation du capteur à la fréquence initiale sous forme d'impulsions d'émission de mesure à la fréquence d'impulsion d'émission de mesure pour une période de mesure ultérieure.
